# EUROPEAN PATENT SPECIFICATION

(11) **EP 3 600 013 B1**
(45) Date of publication and mention of the grant of the patent: **11.03.2026**
(21) Application number: 18771241.9
(22) Date of filing: 15.03.2018
(51) Int. Cl.: A61B 5/00, A61B 5/024, A61B 5/11, A61B 5/0205

(54) **PHYSIOLOGICAL MONITORING DEVICES AND METHODS FOR NOISE REDUCTION IN PHYSIOLOGICAL SIGNALS BASED ON SUBJECT ACTIVITY TYPE**
PHYSIOLOGISCHE ÜBERWACHUNGSVORRICHTUNGEN UND VERFAHREN ZUR RAUSCHVERMINDERUNG BEI PHYSIOLOGISCHEN SIGNALEN AUF DER BASIS DES PERSONENAKTIVITÄTSTYPS
DISPOSITIFS DE SURVEILLANCE PHYSIOLOGIQUE ET PROCÉDÉS DE RÉDUCTION DE BRUIT DANS DES SIGNAUX PHYSIOLOGIQUES SUR LA BASE D'UN TYPE D'ACTIVITÉ DE SUJET

(30) Priority: 23.03.2017 US 201762475746 P
(43) Date of publication of application: 05.02.2020
(73) Proprietor: Yukka Magic LLC, Wilmington DE 19801 (US)
(72) Inventor: AUMER, Michael Edward, Raleigh, North Carolina 27608 (US); LEBOEUF, Steven Francis, Raleigh, North Carolina 27603 (US)
(74) Representative: Bardehle Pagenberg Partnerschaft mbB Patentanwälte Rechtsanwälte
(86) International application number: PCT/US2018/022621
(87) International publication number: WO 2018/175196

(56) References cited:
- EP-A1- 1 908 401
- EP-A2- 2 667 769
- EP-B1- 2 667 769
- WO-A1-2017/027551
- US-A1- 2014 088 433
- US-A1- 2014 213 863
- US-A1- 2014 276 119
- US-A1- 2015 342 527
- US-A1- 2016 089 033
- US-A1- 2016 361 021
- US-A1- 2017 007 166

## Description

### CLAIM OF PRIORITY

This application claims priority from U.S. Provisional Patent Application No. 62/475,746 entitled "PHYSIOLOGICAL MONITORING DEVICES AND METHODS FOR NOISE REDUCTION IN PHYSIOLOGICAL SIGNALS BASED ON SUBJECT ACTIVITY TYPE" filed March 23, 2017.

### FIELD

The present invention relates generally to monitoring devices and methods, more particularly, to monitoring devices and methods for measuring physiological information.

### BACKGROUND

Photoplethysmography (PPG) is based upon shining light into the human body and measuring how the scattered light intensity changes with each pulse of blood flow. The scattered light intensity will change in time with respect to changes in blood flow or blood opacity associated with heart beats, breaths, blood oxygen level (SpO₂), and the like. Such a sensing methodology may require the magnitude of light energy reaching the volume of flesh being interrogated to be steady and consistent so that small changes in the quantity of scattered photons can be attributed to varying blood flow. If the incidental and scattered photon count magnitude changes due to light coupling variation between the source or detector and the skin or other body tissue, then the signal of interest can be difficult to ascertain due to large photon count variability caused by motion artifacts. Changes in the surface area (and volume) of skin or other body tissue being impacted with photons, or varying skin surface curvature reflecting significant portions of the photons may also significantly impact optical coupling efficiency. Physical activity, such a walking, cycling, running, etc., may cause motion artifacts in the optical scatter signal from the body, and time-varying changes in photon intensity due to motion artifacts may swamp-out time-varying changes in photon intensity due to blood flow changes. Each of these changes in optical coupling can dramatically reduce the signal-to-noise ratio (S/N) of biometric PPG information to total time-varying photonic interrogation count. This can result in a much lower accuracy in metrics derived from PPG data, such as heart rate and breathing rate.

An earphone, such as a headset, earbud, etc., may be a good choice for incorporation of a photoplethysmography (PPG) device because it is a form factor that individuals are familiar with, it is a device that is commonly worn for long periods of time, and it is often used during exercise which is a time when individuals may benefit from having accurate heart rate data (or other physiological data). Unfortunately, incorporation of a photoplethysmography device into an earphone poses several challenges. For example, earphones may be uncomfortable to wear for long periods of time, particularly if they deform the ear surface. Moreover, human ear anatomy may vary significantly from person to person, so finding an earbud form that will fit comfortably in many ears may be difficult. Other form-factors for PPG devices, such as wristbands, armbands, clothing, and the like may be problematic for motion artifacts as well.

Some previous efforts to reduce motion artifacts from wearable PPG devices have focused on passive filtering, adaptive filtering (US 8,923,941), or removing noise associated with footsteps (US 8,157,730) with harmonics associated with a subject's cadence (US 2015/0366509). Each of these methods is associated with both strengths and weaknesses, depending on the wearable PPG sensor location or the type of motion noise that is present for the subject wearing the PPG sensor. US 2015/342527 (A1) discloses a further wearable physiological sensor device relevant for understanding the background of the invention. The disclosed device comprises optical pressure sensors 305, used to detect heart rate and wearing pressure, and a motion sensor suite 330, using accelerometers as an example. It fails to disclose selecting a noise reference in response to identification of an activity.

### SUMMARY

It should be appreciated that this Summary is provided to introduce a selection of concepts in a simplified form, the concepts being further described below in the Detailed Description. This Summary is not intended to identify key features or essential features of this disclosure, nor is it intended to limit the scope of the invention. Aspects of the invention provide a monitoring device, a method and a computer program product as claimed in the appended claims.

According to some embodiments of the present invention, a monitoring device configured to be attached to a body of a subject includes a sensor that is configured to detect and/or measure physiological information from the subject (e.g., a PPG sensor, etc.), at least one motion sensor configured to detect and/or measure subject motion information, and at least one processor that is configured to receive and analyze physiological sensor signals produced by the physiological sensor and motion sensor signals produced by the motion sensor. The monitoring device may be configured to be positioned at or within an ear of the subject, secured to an appendage of the subject, or integrated into a form-factor that is wearable at a location of the body of the subject (for example, on a wrist). The physiological sensor may be an optical sensor that includes at least one optical emitter and at least one optical detector, although various other types of sensors may be utilized. The at least one processor is configured to process motion sensor signals to identify an activity characteristic of the subject. For example, the at least one processor is configured to determine if the subject is engaged in a periodic activity such as exercising, running, walking, etc., or if the subject is engaged in a lifestyle or non-periodic activity such as reading a book, desk work, eating, etc. A motion sensor's signal during random motion associated with lifestyle activities will be different than the signal during periodic activities. Once an activity characteristic is determined, the at least one processor is configured to select a noise reference in response to identification of the activity characteristic of the subject, and then process physiological sensor signals using the noise reference to generate output signals having reduced noise relative to the physiological sensor signals and produce physiological information about the subject.

According to the invention, the at least one motion sensor are first and second motion sensors, and selecting the noise reference may include selecting the first motion sensor responsive to identification of the activity characteristic as periodic motion, or selecting the second motion sensor responsive to identification of the activity characteristic as non-periodic motion.

In some embodiments, the physiological sensor may be an optical sensor, and a signal response of the second motion sensor may more closely correspond to motion-related optical noise present in the physiological sensor signal than a signal response of the first motion sensor.

In some embodiments, processing the physiological sensor signal using the noise reference may include processing the physiological sensor signal using a frequency-domain-based algorithm or circuit responsive to identification of the activity characteristic as periodic motion, or processing the physiological sensor signal using a time-domain-based algorithm or circuit responsive to identification of the activity characteristic as non-periodic motion.

In some embodiments, the physiological sensor may be a photoplethysmography (PPG) sensor, the first motion sensor may be an inertial sensor, and wherein the second motion sensor may be an optomechanical sensor.

In some embodiments, processing the physiological sensor signal using the noise reference may further include, in response to identification of the activity characteristic as periodic motion, processing the physiological sensor signal using a first algorithm or circuit, or, in response to identification of the activity characteristic as non-periodic motion, processing the physiological sensor signal using a second algorithm or circuit that is different from the first algorithm or circuit.

In some embodiments, the first algorithm or circuit may be based on frequency-domain processing and/or filtering, and wherein the second algorithm or circuit may be based on time-domain processing and/or filtering.

In some embodiments, the first and second algorithms or circuits may utilize a same sensor or sensors of the at least one motion sensor.

In some embodiments, the first algorithm or circuit may be configured to perform spectral subtraction, and the second algorithm or circuit may be configured to perform adaptive filtering.

In some embodiments, the optomechanical sensor may be configured to modulate light generated by an optical emitter in response to body motion, and to direct the light upon a pathway which is substantially unaffected by blood flow.

In some embodiments, body motion information contained in the noise reference may be greater than blood flow information contained therein.

In some embodiments, the noise reference may include information associated with motion of the subject and may be substantially free of information associated with blood flow of the subject.

In some embodiments, the physiological information may include one or more of the following: subject heart rate, subject respiration rate, subject respiratory rate interval (RRi), subject heart rate variability (HRV), subject blood pressure, subject blood analyte levels, subject cardiovascular properties.

According to some embodiments of the present invention, a monitoring device configured to be attached to a bodily location of a subject includes a physiological sensor configured to detect and/or measure physiological data from the subject and generate a physiological sensor signal representative thereof, at least one motion sensor configured to detect and/or measure subject motion data and generate a motion sensor signal representative thereof, and at least one processor coupled to the physiological sensor and the motion sensor. The at least one processor is configured to perform operations including processing the motion sensor signal received from the at least one motion sensor to identify an activity characteristic of the subject, selecting a noise reference in response to identification of the activity characteristic, and processing the physiological sensor signal received from the physiological sensor using the noise reference to generate an output signal having reduced noise relative to the physiological sensor signal.

According to some embodiments of the present invention, a computer program product includes a non-transitory computer readable storage medium having computer readable program code embodied therein. When executed by at least one processor, the computer readable program code causes the at least one processor to perform operations including processing a motion sensor signal received from at least one motion sensor to identify an activity characteristic of the subject, selecting a noise reference in response to identification of the activity characteristic, and processing a physiological sensor signal received from the physiological sensor using the noise reference to generate an output signal having reduced noise relative to the physiological sensor signal.

Monitoring devices according to embodiments of the present invention can be advantageous over some conventional monitoring devices because, by detecting an activity characteristic of a subject, a corresponding noise reference can be selected and/or modified to reduce noise that may be present in a physiological sensor signal so as to provide more accurate physiological information about a subject. For example, for optimal heart rate tracking accuracy, it may be advantageous to have some context about the type of activity being performed by a subject (i.e., whether the subject is engaged in lifestyle activities or periodic activities) to select a noise reference that may more closely correspond to motion-related noise in the signal from which the heart rate signal.

It is noted that aspects of the invention described with respect to one embodiment may be incorporated in a different embodiment although not specifically described relative thereto. That is, all embodiments and/or features of any embodiment can be combined in any way and/or combination, even if not specifically described in that manner. These and other aspects of the present invention are explained in detail below.

### BRIEF DESCRIPTION OF THE DRAWINGS

The accompanying drawings, which form a part of the specification, illustrate various embodiments of the present invention. The drawings and description together serve to fully explain embodiments of the present invention.
Figs. 1A-1B illustrate a monitoring device that can be positioned within an ear of a subject, according to some embodiments of the present invention.
Fig. 2A illustrates a monitoring device that can be positioned around an appendage (such as an arm, leg, finger, toe, etc.) of the body of a subject, according to some embodiments of the present invention.
Fig. 2B is a cross sectional view of the monitoring device of Fig. 2A.
Fig. 3 is a block diagram of a monitoring device according to some embodiments of the present invention.
Figs. 4-7 are flowcharts of operations for monitoring a subject according to embodiments of the present invention.
Fig. 8 is a graph illustrating heart rate data for a subject plotted over time compared to a benchmark for a range of activity mode settings of a monitoring device, according to some embodiments of the present invention.
Fig. 9 is a graph illustrating heart rate data for a subject plotted over time compared to a benchmark for a range of activity mode settings of a monitoring device, according to some embodiments of the present invention.
Fig. 10 is a spectrogram of accelerometer data showing the subject monitored in Figs. 8 and 9 performing non-periodic activity and periodic activity.
Fig. 11 is a flowchart of operations for selecting a noise reference responsive to monitoring a subject according to embodiments of the present invention.

### DETAILED DESCRIPTION

The present invention will now be described more fully hereinafter with reference to the accompanying figures, in which embodiments of the invention are shown. This invention may, however, be embodied in many different forms and should not be construed as limited to the embodiments set forth herein. Like numbers refer to like elements throughout. In the figures, certain layers, components or features may be exaggerated for clarity, and broken lines illustrate optional features or operations unless specified otherwise. In addition, the sequence of operations (or steps) is not limited to the order presented in the figures and/or claims unless specifically indicated otherwise. Features described with respect to one figure or embodiment can be associated with another embodiment or figure although not specifically described or shown as such.

It will be understood that when a feature or element is referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "secured", "connected", "attached" or "coupled" to another feature or element, it can be directly secured, directly connected, attached or coupled to the other feature or element or intervening features or elements may be present. In contrast, when a feature or element is referred to as being "directly secured", "directly connected", "directly attached" or "directly coupled" to another feature or element, there are no intervening features or elements present. Although described or shown with respect to one embodiment, the features and elements so described or shown can apply to other embodiments.

The terminology used herein is for the purpose of describing particular embodiments only and is not intended to be limiting of the invention. As used herein, the singular forms "a", "an" and "the" are intended to include the plural forms as well, unless the context clearly indicates otherwise.

As used herein, the terms "comprise", "comprising", "comprises", "include", "including", "includes", "have", "has", "having", or variants thereof are open-ended, and include one or more stated features, elements, steps, components or functions but does not preclude the presence or addition of one or more other features, elements, steps, components, functions or groups thereof. Furthermore, as used herein, the common abbreviation "e.g.", which derives from the Latin phrase "exempli gratia," may be used to introduce or specify a general example or examples of a previously mentioned item, and is not intended to be limiting of such item. The common abbreviation "i.e.", which derives from the Latin phrase "id est," may be used to specify a particular item from a more general recitation.

As used herein, the term "and/or" includes any and all combinations of one or more of the associated listed items and may be abbreviated as "/".

As used herein, phrases such as "between X and Y" and "between about X and Y" should be interpreted to include X and Y. As used herein, phrases such as "between about X and Y" mean "between about X and about Y." As used herein, phrases such as "from about X to Y" mean "from about X to about Y."

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

It will be understood that although the terms first and second are used herein to describe various features or elements, these features or elements should not be limited by these terms. These terms are only used to distinguish one feature or element from another feature or element. Thus, a first feature or element discussed below could be termed a second feature or element, and similarly, a second feature or element discussed below could be termed a first feature or element without departing from the teachings of the present invention.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the specification and relevant art and should not be interpreted in an idealized or overly formal sense unless expressly so defined herein. Well-known functions or constructions may not be described in detail for brevity and/or clarity.

The term "about", as used herein with respect to a value or number, means that the value or number can vary more or less, for example by +/- 20%, +/- 10%, +/- 5%, +/- 1%, +/- 0.5%, +/-0.1%, etc.

The terms "sensor", "sensing element", and "sensor module", as used herein, are interchangeable and refer to a sensor element or group of sensor elements that may be utilized to sense information, such as information (e.g., physiological information, body motion, etc.) from the body of a subject and/or environmental information in a vicinity of the subject. A sensor/sensing element/sensor module may comprise one or more of the following: a detector element, an emitter element, a processing element, optics, mechanical support, supporting circuitry, and the like. Both a single sensor element and a collection of sensor elements may be considered a sensor, a sensing element, or a sensor module.

The term "optical emitter", as used herein, may include a single optical emitter and/or a plurality of separate optical emitters that are associated with each other.

The term "optical detector", as used herein, may include a single optical detector and/or a plurality of separate optical detectors that are associated with each other.

The term "wearable sensor module", as used herein, refers to a sensor module configured to be worn on or near the body of a subject.

The terms "monitoring device" and "biometric monitoring device", as used herein, are interchangeable and include any type of device, article, or clothing that may be worn by and/or attached to a subject and that includes at least one sensor/sensing element/sensor module. Exemplary monitoring devices may be embodied in an earpiece, a headpiece, a finger clip, a digit (finger or toe) piece, a limb band (such as an arm band or leg band), an ankle band, a wrist band, a nose piece, a sensor patch, eyewear (such as glasses or shades), apparel (such as a shirt, hat, underwear, etc.), a mouthpiece or tooth piece, contact lenses, or the like.

The term "monitoring" refers to the act of measuring, quantifying, qualifying, estimating, sensing, calculating, interpolating, extrapolating, inferring, deducing, or any combination of these actions. More generally, "monitoring" refers to a way of getting information via one or more sensing elements. For example, "blood health monitoring" includes monitoring blood gas levels, blood hydration, and metabolite/electrolyte levels.

The term "headset", as used herein, is intended to include any type of device or earpiece that may be attached to or near the ear (or ears) of a user and may have various configurations, without limitation. Headsets incorporating optical sensor modules, as described herein, may include mono headsets (a device having only one earbud, one earpiece, etc.) and stereo headsets (a device having two earbuds, two earpieces, etc.), earbuds, hearing aids, ear jewelry, face masks, headbands, and the like. In some embodiments, the term "headset" may include broadly headset elements that are not located on the head but are associated with the headset. For example, in a "medallion" style wireless headset, where the medallion comprises the wireless electronics and the headphones are plugged into or hard-wired into the medallion, the wearable medallion would be considered part of the headset as a whole. Similarly, in some cases, if a mobile phone or other mobile device is intimately associated with a plugged-in headphone, then the term "headset" may refer to the headphone-mobile device combination. The terms "headset" and "earphone", as used herein, are interchangeable.

The term "physiological" refers to matter or energy of or from the body of a creature (e.g., humans, animals, etc.). In embodiments of the present invention, the term "physiological" is intended to be used broadly, covering both physical and psychological matter and energy of or from the body of a creature.

The term "body" refers to the body of a subject (human or animal) that may wear a monitoring device, according to embodiments of the present invention. The term "bodily location" refers to a location of the body on which (or in which) a monitoring device may be worn.

The term "processor" is used broadly to refer to a signal processor or computing system or processing or computing method which may be localized or distributed. For example, a localized signal processor may comprise one or more signal processors or processing methods localized to a general location, such as to a wearable device. Examples of such wearable devices may comprise an earpiece, a headpiece, a finger clip, a digit (finger or toe) piece, a limb band (such as an arm band or leg band), an ankle band, a wrist band, a nose piece, a sensor patch, eyewear (such as glasses or shades), apparel (such as a shirt, hat, underwear, etc.), a mouthpiece or tooth piece, contact lenses, or the like. Examples of a distributed processor comprise "the cloud", the internet, a remote database, a remote processor computer, a plurality of remote processors or computers in communication with each other, or the like, or processing methods distributed amongst one or more of these elements. The key difference is that a distributed processor may include delocalized elements, whereas a localized processor may work independently of a distributed processing system. As a specific example, microprocessors, microcontrollers, ASICs (application specific integrated circuits), analog processing circuitry, or digital signal processors are a few non-limiting examples of physical signal processors that may be found in wearable devices.

The term "remote" does not necessarily mean that a remote device is a wireless device or that it is a long distance away from a device in communication therewith. Rather, the term "remote" is intended to reference a device or system that is distinct from another device or system or that is not substantially reliant on another device or system for core functionality. For example, a computer wired to a wearable device may be considered a remote device, as the two devices are distinct and/or not substantially reliant on each other for core functionality. However, any wireless device (such as a portable device, for example) or system (such as a remote database for example) is considered remote to any other wireless device or system.

The terms "signal analysis frequency" and "signal sampling rate", as used herein, are interchangeable and refer to the number of samples per second (or per other unit) taken from a continuous sensor (i.e., physiological sensor and environmental sensor) signal to ultimately make a discrete signal.

The term "sensor module interrogation power", as used herein, refers to the amount of electrical power required to operate one or more sensors (i.e., physiological sensors and environmental sensors) of a sensor module and/or any processing electronics or circuitry (such as microprocessors and/or analog processing circuitry) associated therewith. Examples of decreasing the sensor interrogation power may include lowering the voltage or current through a sensor element (such as lowering the voltage or current applied to a pair of electrodes), lowering the polling (or polling rate) of a sensor element (such as lowering the frequency at which an optical emitter is flashed on/off in a PPG sensor), lowering the sampling frequency of a stream of data (such as lowering the sampling frequency of the output of an optical detector in a PPG sensor), selecting a lower-power algorithm (such as selecting a power-efficient time-domain processing method for measuring heart rate vs. a more power-hungry frequency-domain processing method), or the like. Lowering the interrogation power may also include powering only one electrode, or powering fewer electrodes, in a sensor module or sensor element such that less total interrogation power is exposed to the body of a subject. For example, lowering the interrogation power of a PPG sensor may comprise illuminating only one light-emitting diode rather than a plurality of light-emitting diodes that may be present in the sensor module, and lowering the interrogation power of a bioimpedance sensor may comprise powering only one electrode pair rather than a plurality of electrodes that may be present in the bioimpedance sensor module.

The term "polling" typically refers to controlling the intensity of an energy emitter of a sensor or to the "polling rate" and/or duty cycle of an energy emitter element in a sensor, such as an optical emitter in a PPG sensor or an ultrasonic driver in an ultrasonic sensor. Polling may also refer to the process of collecting and not collecting sensor data at certain periods of time. For example, a PPG sensor may be "polled" by controlling the intensity of one or more optical emitters, i.e. by pulsing the optical emitter over time. Similarly, the detector of a PPG sensor may be polled by reading data from that sensor only at a certain point in time or at certain intervals, i.e., as in collecting data from the detector of a PPG sensor for a brief period during each optical emitter pulse. A sensor may also be polled by turning on or off one or more elements of that sensor in time, such as when a PPG sensor is polled to alternate between multiple LED wavelengths over time or when an ultrasonic sensor is polled to alternate between mechanical vibration frequencies over time.

The terms "sampling frequency", "signal analysis frequency", and "signal sampling rate", as used herein, are interchangeable and refer to the number of samples per second (or per other unit) taken from a continuous sensor or sensing element (for example, the sampling rate of the thermopile output in a tympanic temperature sensor).

The term "noise reference" refers to a reference information source for an undesired, dynamic noise signal that may be present within a sensor signal. A noise reference signal may be generated by a noise reference source, and this noise reference signal may be processed (i.e., via digital and/or analog electronic processing) to attenuate noise from the sensor signal, yielding a filtered signal that contains cleaner sensor information (i.e., such that the desired sensor information remains with reduced noise). As a specific example, for a PPG sensor worn by a subject, a motion noise reference may include an accelerometer, a pressure sensor, a piezoelectric sensor, a capacitive sensor, an inductive sensor, an optical sensor, or the like. Ideally, the noise reference signal may contain information associated with the motion of the subject, but may contain little (if any) information associated with the blood flow of the subject. Stated another way, the ratio of body motion information to arterial blood flow information contained in the noise reference signal may be relatively high.

Motion noise information generated by the motion noise reference can be processed with PPG sensor data to attenuate dynamically changing motion noise from the PPG sensor, such that the resulting signal contains cleaner information about blood flow (and less motion noise information). In accordance with some embodiments described herein, one motion noise reference for a wearable PPG sensor is an optomechanical sensor also known as a "blocked channel" sensor. This optomechanical sensor can be designed or configured to modulate light generated by an optical emitter in response to body motion, and to direct that light upon a pathway that does not interact with blood flow; in this manner, the optomechanical sensor can be configured to measure body motion and not blood flow motion, so that the noise reference signal may be used to filter out motion noise from the PPG signal with analog or digital signal processing (i.e. filtering). Nonlimiting examples of such noise references are presented in commonly-owned U.S. Patent No. 9,289,175 entitled *"LIGHT-GUIDING DEVICES AND MONITORING DEVICES INCORPORATING SAME,"* and International Patent Application No. PCT/US2016/046273 entitled *"METHODS AND APPARATUS FOR DETECTING MOTION VIA OPTOMECHANICS'.*

It should be noted that "algorithm" and "circuit" are referred to herein. An algorithm refers to an instruction set, such as an instruction set with sequential steps and logic, that may be in memory whereas a circuit refers to electronic components and/or traces that may implement such logic operations.

It should be noted that processes for managing hysteresis are implied herein. Namely, several embodiments herein for controlling sensors (and other wearable hardware) may involve a processor sending commands to a sensor element depending on the sensor readings. Thus, in some embodiments, a sensor reading (such as a reading from an optical detector or a sensing electrode) above X may result in a processor sending a command to electrically bias another sensor element (such as an optical emitter or a biasing electrode) above Y. Similarly, as soon as the sensor reading drops below X, a processor may send a command to bias another sensor element below Y. However, in borderline situations this may cause unwanted hysteresis in the biasing command, as sensor readings may rapidly toggle above/below X resulting in the toggling of the biasing of another sensor element above/below Y. As such, hysteresis management may be integrated within the algorithm(s) for controlling the execution of a processor. For example, the processor may be configured by the algorithm to delay a biasing command by a period of time Z following the timing of a prior biasing command, thereby preventing or reducing the aforementioned toggling.

In the following figures, various monitoring devices will be illustrated and described for attachment to the ear or an appendage of the human body. However, it is to be understood that embodiments of the present invention are not limited to those worn by humans.

The ear is an ideal location for wearable health and environmental monitors. The ear is a relatively immobile platform that does not obstruct a person's movement or vision. Monitoring devices located at an ear have, for example, access to the inner-ear canal and tympanic membrane (for measuring core body temperature), muscle tissue (for monitoring muscle tension), the pinna, earlobe, and elsewhere (for monitoring blood gas levels), the region behind the ear (for measuring skin temperature and galvanic skin response), and the internal carotid artery (for measuring cardiopulmonary functioning), etc. Virtually all areas of the ear support enough blood perfusion to enable an assessment of blood flow and blood-flow-derived metrics (such as breathing rate, heart rate, blood pressure, RR-interval, and the like) via photoplethysmography (PPG). A particularly good location for PPG may be the region covering the antitragus and concha regions of the ear, as the region has high perfusion, is more resistant to motion artifacts, and is in a region away from the ear canal. The ear is also at or near the point of exposure to: environmental breathable toxicants of interest (volatile organic compounds, pollution, etc.); noise pollution experienced by the ear; and lighting conditions for the eye. Furthermore, as the ear canal is naturally designed for transmitting acoustical energy, the ear provides a good location for monitoring internal sounds, such as heartbeat, breathing rate, and mouth motion.

Optical coupling into the blood vessels of the ear may vary between individuals. As used herein, the term "coupling" refers to the interaction or communication between excitation energy (such as light) entering a region and the region itself. For example, one form of optical coupling may be the interaction between excitation light generated from within an optical sensor of a wearable device -- such as a wristband, armband, legband, skin-worn patch, neckband, ring, earbud, other device positioned at or within an ear, a headband, or the like -- and the blood vessels. In one embodiment, this interaction may involve excitation light entering the skin and scattering from a blood vessel such that the temporal change in intensity of scattered light is proportional to a temporal change in blood flow within the blood vessel. Another form of optical coupling may be the interaction between excitation light generated by an optical emitter within a wearable device and a light-guiding region of the wearable device. Thus, a wearable device with integrated light-guiding capabilities, wherein light can be guided to multiple and/or select regions along the wearable device, can assure that each individual wearing the device will generate an optical signal related to blood flow through the blood vessels. Optical coupling of light to a particular skin region of one person may not yield photoplethysmographic signals for each person. Therefore, coupling light to multiple regions may assure that at least one blood-vessel-rich region will be interrogated for each person wearing the device. Coupling multiple regions of the body to light may also be accomplished by diffusing light from a light source in the device.

According to some embodiments of the present invention, "smart" monitoring devices including, but not limited to, wrist-worn devices, armbands, earbuds, and the like, are provided that can modify biometric signal extraction algorithms based upon a recognition of an activity level of a subject.

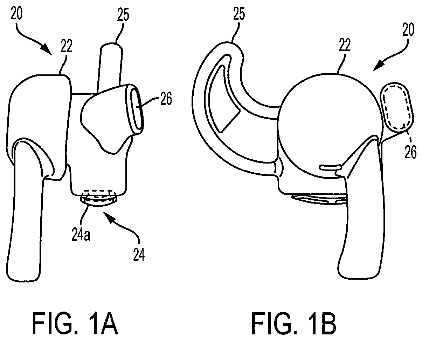

Figs. 1A-1B illustrate a monitoring apparatus 20 configured to be positioned within an ear of a subject, according to some embodiments of the present invention. The illustrated apparatus 20 includes an earpiece body or housing 22, a sensor module 24, a stabilizer 25, and a sound port 26. When positioned within the ear of a subject, the sensor module 24 has a region 24a configured to contact a selected area of the ear. The illustrated sensor region 24a is contoured (i.e., is "form-fitted") to matingly engage a portion of the ear between the anti tragus and acoustic meatus, and the stabilizer is configured to engage the anti-helix. However, monitoring devices in accordance with embodiments of the present invention can have sensor modules with one or more regions configured to engage various portions of the ear. Various types of device configured to be worn at or near the ear may be utilized in conjunction with embodiments of the present invention.

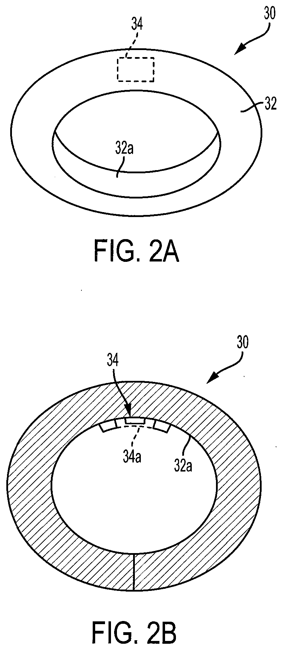

Figs. 2A-2B illustrate a monitoring apparatus 30 in the form of a sensor band 32 configured to be secured to an appendage (e.g., an arm, wrist, hand, finger, toe, leg, foot, neck, etc.) of a subject. The band 32 includes a sensor module 34 on or extending from the inside surface 32a of the band 32. The sensor module 34 is configured to detect and/or measure physiological information from the subject and includes a sensor region 34a that is contoured to contact the skin of a subject wearing the apparatus 30.

Embodiments of the present invention may be utilized in various devices and articles including, but not limited to, patches, clothing, etc. Embodiments of the present invention can be utilized wherever PPG and blood flow signals can be obtained and at any location on the body of a subject. Embodiments of the present invention are not limited to the illustrated monitoring devices 20, 30 of Figs. 1A-1B and 2A-2B.

The sensor modules 24, 34 for the illustrated monitoring devices 20, 30 of Figs. 1A-1B and 2A-2B are configured to detect and/or measure physiological information from a subject wearing the monitoring devices 20, 30. In some embodiments, the sensor modules 24, 34 may be configured to detect and/or measure one or more environmental conditions in a vicinity of the subject wearing the monitoring devices 20, 30.

A sensor module utilized in accordance with embodiments of the present invention may be an optical sensor module that includes at least one optical emitter and at least one optical detector. Exemplary optical emitters include, but are not limited to light-emitting diodes (LEDs), laser diodes (LDs), compact incandescent bulbs, micro-plasma emitters, IR blackbody sources, or the like. In addition, a sensor module may include various types of sensors including and/or in addition to optical sensors. For example, a sensor module may include one or more inertial sensors (e.g., an accelerometer, piezoelectric sensor, vibration sensor, photoreflector sensor, etc.) for detecting changes in motion, one or more thermal sensors (e.g., a thermopile, thermistor, resistor, etc.) for measuring temperature of a part of the body, one or more electrical sensors for measuring changes in electrical conduction, one or more skin humidity sensors, and/or one or more acoustical sensors. Sensor modules may include physiological sensors for monitoring vital signs, blood flow properties, body fluids, muscle motion, body motion, and the like, Sensor modules may include environmental sensors for monitoring ambient light, ambient temperature, ambient humidity, airborne pollutants, wind, and the like. It should be understood that a wide variety of sensors may be incorporated into a wearable sensor module.

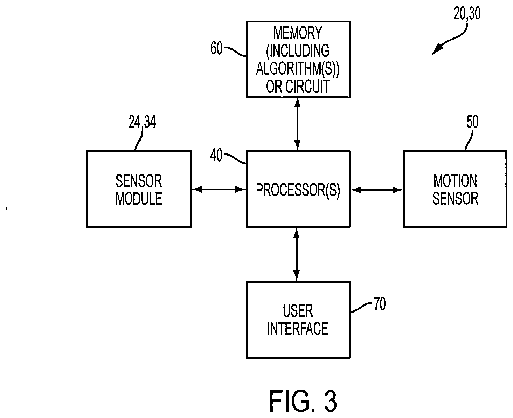

Referring to Fig. 3, a monitoring device (e.g., monitoring devices 20, 30), according to embodiments of the present invention, is illustrated schematically. The illustrated monitoring device includes a sensor module 24, 34 having one or more physiological sensors configured to detect and/or measure physiological information from the subject, and a motion sensor 50 (e.g., an accelerometer) configured to detect and/or measure subject motion information. The monitoring device 20, 30 also includes at least one processor 40 that is coupled to the sensor(s) of a sensor module 24, 34, to the motion sensor 50, and that is configured to receive and analyze signals produced by the sensor(s) and motion sensor 50.

According to some embodiments of the present invention, the processor 40 is configured to process motion sensor signals to identify an activity characteristic (e.g., periodic or non-periodic activity) of the subject, select or modify a biometric signal extraction algorithm (implemented by computer-readable memory and/or circuit 60) in response to identification of the type of subject activity, and then process physiological sensor signals via the modified biometric signal extraction algorithm or circuit 60 to produce physiological information about the subject, such as subject heart rate, subject respiration rate, subject RRi (i.e., the time-difference between consecutive R-peaks in the ECG or PPG waveform), subject HRV, subject blood pressure, subject blood analyte levels, subject cardiovascular properties, and the like. Although many aspects of this invention are most related to PPG sensors, a variety of other physiological sensors may be used within the scope of this invention.

In some embodiments, the motion sensor 50 is an accelerometer and a filter is applied to each axis of the accelerometer data to remove the DC component therefrom. A spectral transform is performed on each axis of the DC-removed signal over a given time span. An accelerometer spectrum is then generated by taking the root of the sum of the squares of each axis of the accelerometer that is associated with that time span. Other techniques may also be used.

The 1st, 2nd, and 3rd, most intense frequencies and magnitudes in the accelerometer spectrum are identified. The relative proportion of energy within the peaks is compared to all other frequencies to determine if the criteria for periodicity is met. If so, the given segment of time is determined to represent periodic activity and an Automatic Mode Switching feature of the monitoring device is set to inform an algorithm 60 (e.g., a heart rate algorithm, etc.) of the periodic context. If not, an Automatic Mode Switching feature of the monitoring device is set to inform the HR algorithm of the non-periodic context.

Alternatively or in addition, a determination is made whether the ratio of peak frequencies fit a pattern associated with periodicity (e.g., whether peak 2 is twice the frequency of peak 1, etc.). If so, the given segment of time is determined to represent periodic activity and an Automatic Mode Switching feature of the monitoring device is set to inform an algorithm 60 (e.g., a heart rate algorithm, etc.) of the periodic context. If not, an Automatic Mode Switching feature is set to inform the HR algorithm of the non-periodic context.

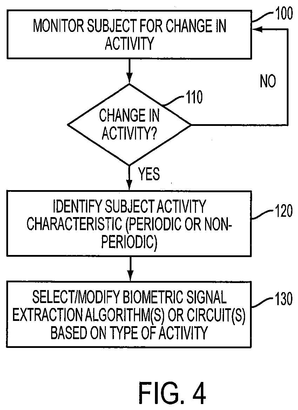

Referring now to Fig. 4, a method of monitoring a subject via a monitoring device, such as monitoring devices 20, 30, according to some embodiments of the present invention, will be described. The monitoring device includes a physiological sensor configured to detect and/or measure physiological information from the subject and a motion sensor. The physiological sensor and the motion sensor are in communication with a processor that is configured to receive and analyze signals produced by the physiological sensor and motion sensor. The processor may be part of the monitoring device or may be a remotely located processor.

The subject is monitored for a change in physical activity level or type (Block 100). If a change is detected (Block 110), the processor 40 identifies an activity characteristic (e.g., determines if the activity is periodic or non-periodic) (Block 120). For example, via an algorithm stored in or implemented by memory or circuit 60, the processor determines if the subject is engaged in a non-periodic or lifestyle activity such as reading a book, desk work, eating, etc., or a periodic activity such as exercising, running, walking, etc. In response to determining the type of activity, the processing of the biometric signal extraction algorithm may be modified in one or more ways (Block 130). For example, in response to determining the type of activity, at least one parameter of the biometric signal extraction algorithm may be modified, and this modified biometric signal extraction algorithm may be selected for processing. Some specific examples of parameters that may be modified are described in US 2015/0366509 entitled "CADENCE DETECTION BASED ON INERTIAL HARMONICS' (published December 24, 2015), US 2016/0029964 entitled "PHYSIOLOLOGICAL MONITORING DEVICES WITH ADJUSTABLE SIGNAL ANALYSIS AND INTERROGATION POWER AND MONITORING METHODS USING THE SAME " (published February 4, 2016), and US 2015/0011898 entitled "PHYSIOLOLOGICAL METRIC ESTIMATION RISE AND FALL LIMITING" (published January 8, 2015). For example, parameters affecting the rise or fall time of biometric tracking (such as the tracking of heart rate, breathing rate, RRi, etc.), parameters affecting the subtraction or redaction of unwanted frequencies in the PPG signal (i.e., which frequency bands to subtract or redact (set to zero) based for example on amplitude and/or power), and/or parameters affecting which types of filters to execute (highpass, lowpass, bandpass, active, subtractive or redactive filters, or the like) may be modified. In response to determining the type of activity, the processor may thus select a biometric signal extraction algorithm (which may be all or part of algorithm stored in or implemented by memory or circuit 60) so as to be best suited for processing sensor data during such activity (Block 130) (i.e., that is best suited for removing motion or environmental noise from that particular activity).

It should be noted that, in addition to or alternatively than modifying an algorithm or selecting an algorithm, a circuit may be modified or selected. For example, the determination of periodicity in Block 120 may send a signal to one or more transistors (Block 130) to switch the analog filtering circuits to "notch out" the periodic signal; or, the determination of non-periodicity in Block 120 may send a signal to one or more transistors (Block 130) to switch the analog filtering circuits to a bandpass filter that includes the biometric signals of interest. It should also be noted that both algorithms and circuits may simultaneously be modified or selected in the embodiments described herein. Thus, as another exemplary embodiment, the determination of periodicity in Block 120, using a low-power time-domain algorithm (such as an autocorrelation function, or the like), may initiate an algorithm in Block 130 that determines which frequencies should be removed. The output of the algorithm (i.e., the output from a processor) may send a signal to a bank of analog filters in Block 130 to remove these frequencies without the need for digital removal. This may be advantageous because executing a spectral transform may be overly power-hungry in a wearable device. Physiological sensor signals are then processed via the selected biometric signal extraction algorithm to produce physiological information about the subject, such as subject heart rate, subject respiration rate, subject RRi, subject HRV, subject blood pressure, subject blood analyte levels, subject cardiovascular properties, and the like.

Identification of an activity characteristic (e.g., determining if the activity is periodic or non-periodic) may also be associated with particular metrics to be assessed. For example, detecting that periodic motion exists may be a criteria for determining that a cardiac efficiency metric is being assessed. In contrast, if the subject's motion is aperiodic, then a cardiac efficiency score (Average Cadence/Average HR) may be meaningless. Also, one or more processors or functions may be deactivated or run in power-saving mode based on the detection of type of motion (e.g., a pedometer may be deactivated responsive to detection of aperiodic motion). Additionally, an energy expenditure calculation which factors both HR and footsteps into an equation for the estimation of calories burned may be tuned to detect "fidgeting" (subtle motions) rather than footsteps and update the energy expenditure model to incorporate fidgets and HR, rather than footsteps and HR. The weighting factors for fidgets and HR, for example, may be different than the weighting factors for footsteps and HR when estimating energy expenditure.

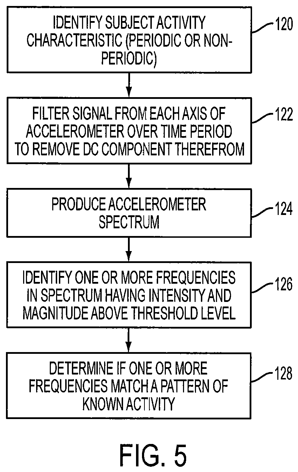

As illustrated in Fig. 5, identifying an activity characteristic of a subject (Block 120) may include filtering a signal from each axis of the accelerometer over a given time period to remove a DC component therefrom (Block 122), producing an accelerometer spectrum using the filtered signals (Block 124), identifying one or more frequencies in the spectrum having a magnitude above a threshold level (Block 126), and/or determining if the one or more frequencies in the spectrum match a pattern associated with a known subject activity mode (Block 128). It should be noted that the operations of Fig. 5 may be controlled by algorithms, circuitry, or a combination of both.

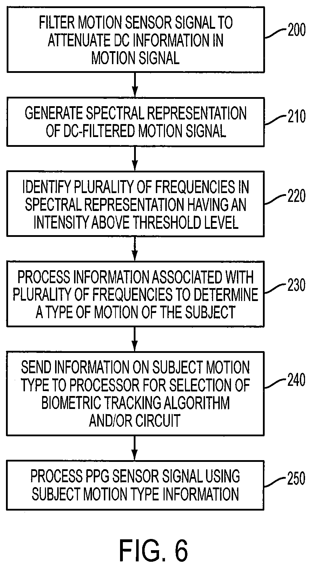

Referring now to Fig. 6, a method of monitoring a subject via a monitoring device, such as monitoring devices 20, 30, according to some embodiments of the present invention, will be described. The monitoring device includes a PPG sensor and a motion sensor which are in communication with a processor that is configured to receive and analyze signals produced by the sensor module and motion sensor. The processor may be part of the monitoring device or may be a remotely located processor. It should be noted that the operations of Fig. 6 may be controlled by algorithms, circuitry, or a combination of both.

The method includes filtering a motion signal generated by the motion sensor to attenuate DC information in the motion signal and generate a DC-filtered motion signal (Block 200). A spectral representation of the DC-filtered motion signal is generated (Block 210) and a plurality of frequencies in the spectral representation are identified having a magnitude above a threshold level (Block 220). In other words, the most intense frequencies in the spectral representation are identified. Additional operations may include filtering a step rate component from a measured heart rate component based on a function of a difference therebetween as described in US 2015/0018636 entitled "REDUCTION OF PHYSIOLOGICAL METRIC ERROR DUE TO INERTIAL CADENCE" (published January 15, 2015), comparing frequency measurements from an inertial sensor with one or more thresholds as described in US 2015/0366509 entitled "CADENCE DETECTION BASED ON INERTIAL HARMONICS" (published December 24, 2015), and measuring signal quality to determine how a user should adjust a monitoring device to improve biometric fit, as described in US 2016/0094899 entitled "METHODS AND APPARATUS FOR IMPROVING SIGNAL QUALITY IN WEARABLE BIOMETRIC MONITORING DEVICES" (published March 31, 2016), individually or together in combination. Information associated with the plurality of frequencies is then processed to determine a type of motion of the subject, for example, whether the subject motion is periodic or non-periodic motion (Block 230). Information on the subject motion type is sent to a biometric tracking algorithm or circuit or is otherwise used by the processor to select a biometric tracking algorithm or circuit (Block 240), and a PPG sensor signal is processed using the biometric tracking algorithm or circuit corresponding to the subject motion type information to generate biometric information about the subject (Block 250). Exemplary biometric information includes, but is not limited to, subject heart rate, subject respiration rate, subject RRi, subject HRV, subject blood pressure, subject blood analyte levels, subject cardiovascular properties, etc.

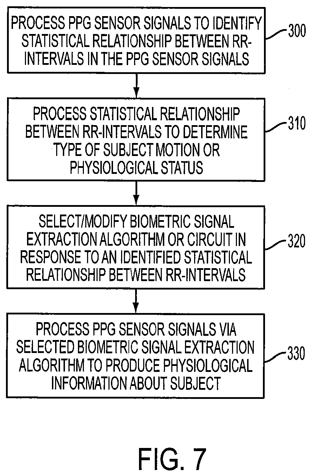

Referring now to Fig. 7, a method of monitoring a subject via a monitoring device, such as monitoring devices 20, 30, according to some embodiments of the present invention, will be described. The monitoring device includes a PPG sensor and a motion sensor which are in communication with a processor that is configured to receive and analyze signals produced by the sensor module and motion sensor. The processor may be part of the monitoring device or may be a remotely located processor. It should be noted that the operations of Fig. 7 may be controlled by algorithms, circuitry, or a combination of both.

The method includes processing PPG sensor signals via the at least one processor to identify a statistical relationship between RR-intervals in the PPG sensor signals (Block 300) and processing the statistical relationship between RR-intervals to determine a type of motion of the subject (e.g., periodic or non-periodic) or a physiological status of the subject (Block 310). A biometric signal extraction algorithm or circuit is modified or selected via the processor in response to an identified statistical relationship between RR-intervals (Block 320). PPG sensor signals are then processed via the selected biometric signal extraction algorithm to produce physiological information about the subject (Block 320), such as subject heart rate, subject respiration rate, subject RR-interval (RRi), subject HRV, subject blood pressure, subject blood analyte levels, subject cardiovascular properties, etc. A particular example of processing RR-intervals to determine a type of motion of the subject may include processing a series of RR-intervals to identify the existence of stress, which may be associated with aperiodic (non-periodic) motion.

It should be noted that it is known to those skilled in the art that a series of RR-intervals may be processed to generate an assessment of fatigue, stress (particularly psychosocial stress), autonomic nervous functioning, hemodynamics, cardiac disease - such as atrial fibrillation or cardiac arrhythmia - or the like. However, the inventors have discovered that the statistical analysis alone may not be sufficient to generate sufficient physiological information for a diagnosis of a health condition or other physiological state. Thus, in one particular embodiment of Fig. 7, the RRi-intervals may be statistically processed (Block 310) to generate an assessment that a subject status of atrial fibrillation is likely. Once this status is known to be likely, the signal extraction algorithm may be modified or selected to more concretely determine a status of atrial fibrillation or to diagnose another condition (such as poor or abnormal hemodynamics or cardiac output, which may be associated with atrial fibrillation). As a specific example, Block 320 may switch to an algorithm that allows more frequencies to pass through during signal extraction, such that the extracted signal from Block 320 may be further processed by Block 330 to generate sufficient physiological information to diagnose atrial fibrillation. It should be understood that this describes a particular embodiment, and other embodiments that rely on Fig. 7 may be used with the present invention. As another specific example, Block 310 may determine that a subject may likely be in a status of fatigue. Block 320 may then modify or select a signal extraction algorithm that increases the signal resolution (i.e., the sampling rate and/or dynamic range), such that the extracted signal may be processed by Block 330 to generate a more definitive assessment of fatigue. In yet another example, Block 310 may determine that a subject may likely be in a status of stress, Block 320 may then modify or select a signal extraction algorithm that switches from a screening spectral-based (frequency domain-based) RRi calculator to a higher resolution time-domain-based RRi calculator, the extracted RRi signal may then be processed by Block 330 to generate a more definitive assessment of stress.

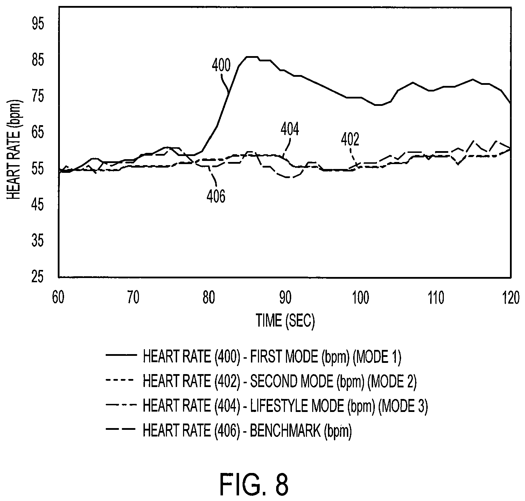

Fig. 8 is a graph illustrating heart rate data 400, 402, 404 for a subject plotted over time compared to the actual heart rate (406), the heart rate determined by a validated benchmark device, for a range of activity mode settings for a monitoring device according to some embodiments of the present invention. For the illustrated duration, the subject was not walking/jogging/running (i.e., the subject was not engaged in a periodic activity), but was doing deskwork (i.e., the subject was engaged in a non-periodic activity), where arms and hands were moving as is typical for desk work. As seen, the heart rate in Mode 3 (the "lifestyle mode" indicated by 404) most closely agrees with that of the benchmark device heart rate (406), and the monitoring device correctly uses the output for Mode 3.

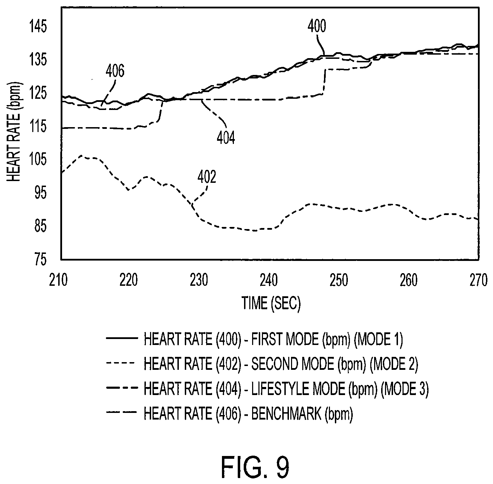

Fig. 9 is a graph illustrating heart rate data 400, 402, 404 for a subject plotted over time compared to the actual heart rate (406), the heart rate determined by a validated benchmark device, for a range of activity mode settings for a monitoring device according to some embodiments of the present invention. For the illustrated duration, the subject is jogging. As seen, the heart rate in Mode 1 (400) most closely agrees with that of the benchmark device heart rate (406), and the monitoring device correctly uses the output for Mode 1. Note that the heart rate reported using Lifestyle mode (404) does not match the benchmark (406) as well as Mode 1 output (400).

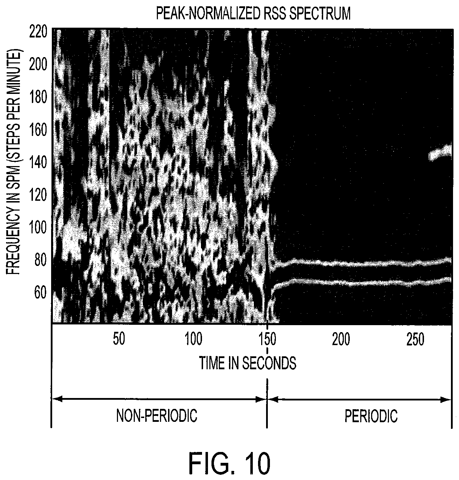

Fig. 10 is a spectrogram for exemplary combined accelerometer data showing the subject monitored in Figs. 8 and 9 performing non-periodic activity from time (t)=0 to 150 seconds, and performing periodic activity (walking) after t=150 seconds. For accurate heart rate tracking, the monitoring device uses settings associated with Lifestyle Mode (Mode 3) prior to t=150 sec and Periodic Mode after t=120 seconds.

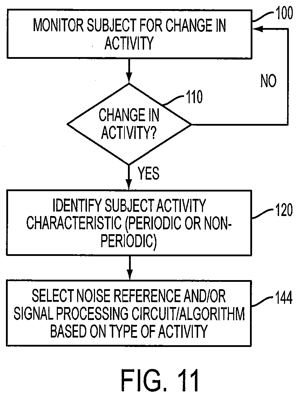

Returning to Fig. 4, a method of monitoring a subject via a monitoring device, block 130 may be modified in accordance with additional embodiments based on the detection of periodic or non-periodic motion. For example, in one embodiment shown as Block 144 in Fig 11, if the processor 40 identifies an activity characteristic (e.g., determines if the activity is periodic or non-periodic) (Block 120) responsive to monitoring a subject (Block 100) and detecting a change in activity (Block 110), then a particular noise reference or set of noise references may be selected for improved (or optimal) motion noise reduction (or removal) from a physiological signal (Block 144). As a specific example for a PPG sensor (such as that similar to that of Fig. 2 and/or Fig. 3) worn by a subject, the PPG sensor having both optical sensors and a plurality of motion sensors, it may be desirable to select a particular type of motion sensor (or a particular set of motion sensors) to remove periodic noise and to select a different type of motion sensor (or a set of different motion sensors) to remove aperiodic noise. For example, upon determining at Block 120 that the activity characteristic is periodic, in Block 144 an accelerometer may be selected as the noise reference. As with descriptions of Fig. 4, this selection may be controlled by one or more algorithms on a microprocessor and/or electronic circuitry. In contrast, upon detecting that the activity characteristic is aperiodic, an optomechanical sensor, such as "blocked channel sensor", may be selected as the noise reference.

One reason why an accelerometer may be a more suitable noise reference for periodic motion over an optomechanical sensor is because an accelerometer may have a better dynamic range or may have a stronger signal response at intense motions associated with periodic motion (such as running, sprinting, cycling, or the like). In contrast, an optomechanical sensor may have a better sensitivity and/or resolution at less intense motion levels (such as resting, walking, typing, grabbing, or the like) or may have a stronger signal response at less intense motions associated with aperiodic motion. Moreover, for a PPG sensor, because the signal response of an optomechanical noise reference may more closely resemble or otherwise correspond to the unwanted motion-related optical noise from the PPG sensor signal, the PPG and optomechanical sensors are likely to be more linear with each other than would be the paring of a PPG sensor and inertial noise reference (such as an accelerometer). Thus, time-domain-based PPG motion noise removal methods or algorithms (such as adaptive filtering and the like), often well-suited for aperiodic motion, may be more effective with this high linearity. In contrast, frequency-domain-based PPG motion noise removal methods or algorithms (such as spectral subtraction and the like), often well-suited for periodic motion, may not be as sensitive to the linearity between the PPG sensor and the noise reference and may work more effectively with an inertial noise reference.

Considering a pressure sensor as an example of a "motion sensor" in Fig. 4 and Fig. 11, in one embodiment a detection of aperiodic motion may result in an accelerometer being deselected as the noise reference and a pressure sensor being selected as a noise reference (or a combination of a pressure sensor and an accelerometer). This is because (as with an optomechanical sensor) a pressure sensor may be more sensitive to aperiodic motion than an inertial sensor but may also be less effective at sensing periodic motion. In some embodiments, Block 144 may not change the noise reference in response to Block 120 but rather may change the algorithm or circuit for noise reduction or removal using the same noise reference. As a specific example, the detection of periodic motion (Block 120) may trigger a spectral noise (frequency domain) reduction or removal algorithm using an accelerometer as a noise reference (Block 130), but the detection of aperiodic motion may trigger a time-domain noise reduction or removal algorithm using the same accelerometer as a noise reference. This may be beneficial because spectral noise reduction or removal algorithms may be more suited for periodic activities (especially where "cross-overs" between step rate and heart rate are observed) and time-domain noise reduction or removal algorithms may be more suited for aperiodic activities (where "cross-overs" are much less likely).

Example embodiments are described herein with reference to block diagrams and flowchart illustrations. It is understood that a block of the block diagrams and flowchart illustrations, and combinations of blocks in the block diagrams and flowchart illustrations, can be implemented by computer program instructions that are performed by one or more computer circuits. These computer program instructions may be provided to a processor circuit of a general purpose computer circuit, special purpose computer circuit, and/or other programmable data processing circuit to produce a machine, such that the instructions, which execute via the processor of the computer and/or other programmable data processing apparatus, transform and control transistors, values stored in memory locations, and other hardware components within such circuitry to implement the functions/acts specified in the block diagrams and flowchart block or blocks, and thereby create means (functionality) and/or structure for implementing the functions/acts specified in the block diagrams and flowchart blocks.

These computer program instructions may also be stored in a tangible computer-readable medium that can direct a computer or other programmable data processing apparatus to function in a particular manner, such that the instructions stored in the computer-readable medium produce an article of manufacture including instructions which implement the functions/acts specified in the block diagrams and flowchart blocks.

A tangible, non-transitory computer-readable medium may include an electronic, magnetic, optical, electromagnetic, or semiconductor data storage system, apparatus, or device. More specific examples of the computer-readable medium would include the following: a portable computer diskette, a random access memory (RAM) circuit, a read-only memory (ROM) circuit, an erasable programmable read-only memory (EPROM or Flash memory) circuit, a portable compact disc read-only memory (CD-ROM), and a portable digital video disc read-only memory (DVD/Blu-Ray).

The computer program instructions may also be loaded onto a computer and/or other programmable data processing apparatus to cause a series of operational steps to be performed on the computer and/or other programmable apparatus to produce a computer-implemented process such that the instructions which execute on the computer or other programmable apparatus provide steps for implementing the functions/acts specified in the block diagrams and flowchart blocks. Accordingly, embodiments of the present invention may be embodied in hardware and/or in software (including firmware, resident software, micro-code, etc.) that runs on a processor such as a digital signal processor, which may collectively be referred to as "circuitry," "a module" or variants thereof.

It should also be noted that in some alternate implementations, the functions/acts noted in the blocks may occur out of the order noted in the flowcharts. For example, two blocks shown in succession may in fact be executed substantially concurrently or the blocks may sometimes be executed in the reverse order, depending upon the functionality/acts involved. Moreover, the functionality of a given block of the flowcharts and block diagrams may be separated into multiple blocks and/or the functionality of two or more blocks of the flowcharts and block diagrams may be at least partially integrated. Finally, other blocks may be added/inserted between the blocks that are illustrated. Moreover, although some of the diagrams include arrows on communication paths to show a primary direction of communication, it is to be understood that communication may occur in the opposite direction to the depicted arrows.

The foregoing is illustrative of the present invention and is not to be construed as limiting thereof. Although a few exemplary embodiments of this invention have been described, those skilled in the art will readily appreciate that many modifications are possible in the exemplary embodiments without materially departing from the teachings and advantages of this disclosure. The invention is defined by the following claims.

## Claims

1. A monitoring device (20, 30) configured to be attached to a bodily location of a subject, the monitoring device comprising:
a sensor module (24, 34) having:
one or more physiological sensors configured to detect and/or measure physiological data from the subject and generate a physiological sensor signal representative thereof;
first and second motion sensors (50) configured to detect and/or measure subject motion data and generate a motion sensor signal representative thereof, wherein the first motion sensor comprises an accelerometer and the second motion sensor comprises either an optomechanical sensor or a pressure sensor; and
at least one processor (40) coupled to the one or more physiological sensors (24, 34) and the first and second motion sensors (50),
wherein
the at least one processor (40) is configured to perform operations comprising:
processing the motion sensor signal received from the first and second motion sensors (50) to identify an activity characteristic of the subject;
selecting a noise reference in response to identification of the activity characteristic; and
processing the physiological sensor signal received from the one or more physiological sensors (24, 34) using the noise reference to generate an output signal having reduced noise relative to the physiological sensor signal.

2. The monitoring device of Claim 1, wherein selecting the first motion sensor or the second motion sensor as a noise reference comprises:
selecting the first motion sensor responsive to identification of the activity characteristic as periodic motion; or
selecting the second motion sensor responsive to identification of the activity characteristic as non-periodic motion.

3. The monitoring device of Claim 2, wherein the physiological sensors (24, 34) comprise an optical sensor, and wherein a signal response of the second motion sensor more closely corresponds to motion-related optical noise present in the physiological sensor signal than a signal response of the first motion sensor (50).

4. The monitoring device of Claims 2 or 3, wherein the one or more physiological sensors (24, 34) include a photoplethysmography, PPG, sensor.

5. The monitoring device of Claim 4, wherein the optomechanical sensor (50) is configured to modulate light generated by an optical emitter in response to body motion, and to direct the light upon a pathway which is substantially unaffected by blood flow.

6. The monitoring device of any preceding claim wherein processing the physiological sensor signal using the noise reference comprises:
processing the physiological sensor signal using a frequency-domain-based algorithm or circuit responsive to identification of the activity characteristic as periodic motion; or
processing the physiological sensor signal using a time-domain-based algorithm or circuit responsive to identification of the activity characteristic as non- periodic motion.

7. The monitoring device of Claim 1, wherein processing the
physiological sensor signal using the noise reference further comprises:
in response to identification of the activity characteristic as periodic motion, processing the physiological sensor signal using a first algorithm or circuit; or
in response to identification of the activity characteristic as non-periodic motion, processing the physiological sensor signal using a second algorithm or circuit that is different from the first algorithm or circuit.

8. The monitoring device of Claim 7, wherein the first and second algorithms or circuits utilize a same sensor or sensors of the at least one motion sensor.

9. The monitoring device of Claims 7 or 8, wherein the first algorithm or circuit is configured to perform spectral subtraction, and wherein the second algorithm or circuit is configured to perform adaptive filtering.

10. The monitoring device of Claim 1, wherein body motion information contained in the noise reference is greater than blood flow information contained therein.

11. The monitoring device of Claim 1, wherein the noise reference comprises information associated with motion of the subject and is substantially free of information associated with blood flow of the subject.

12. The monitoring device of Claim 1, wherein the physiological information includes one or more of the following: subject heart rate, subject respiration rate, subject respiratory rate interval, RRi, subject heart rate variability, HRV, subject blood pressure, subject blood analyte levels, subject cardiovascular properties.

13. The monitoring device of Claim 1, wherein the activity characteristic comprises a first activity characteristic or a second activity characteristic that is different than the first activity characteristic, wherein selecting the noise reference comprises:
selecting the first motion sensor (50) responsive to identification of the first activity characteristic; or
selecting the second motion sensor (50) responsive to identification of the second activity characteristic.

14. A method of monitoring a subject via a monitoring device (20, 30), wherein the monitoring device includes a sensor module (24, 34) having one or more physiological sensors configured to detect and/or measure physiological data from the subject and generate a physiological sensor signal representative thereof, and first and second motion sensors (50) each configured to detect and/or measure subject motion data and to generate a motion sensor signal representative thereof, wherein the first motion sensor comprises an accelerometer and the second motion sensor comprises either an optomechanical sensor or a pressure sensor, and wherein the physiological sensor and the at least one motion sensor are in communication with at least one processor (40), the method comprising:
processing a motion sensor signal received from at least one of the first and second motion sensors (50) by the at least one processor (40) to identify an activity characteristic of the subject;
selecting the first motion sensor or the second motion sensor as a noise reference by the at least one processor (40) in response to identification of the activity characteristic; and
processing a physiological sensor signal received from the physiological sensor (24, 34) by the at least one processor (40) using the noise reference to generate an output signal having reduced noise relative to the physiological sensor signal.

15. A computer program product comprising a non-transitory computer readable storage medium (60) having computer readable program code embodied therein, which, when executed by at least one processor (40), causes the at least one processor to perform operations for a method of monitoring a subject via a monitoring device (20, 30), wherein the monitoring device includes a sensor module (24, 34) having one or more physiological sensors configured to detect and/or measure physiological data from the subject and generate a physiological sensor signal representative thereof, and first and second motion sensors (50) each configured to detect and/or measure subject motion data and to generate a motion sensor signal representative thereof, wherein the first motion sensor comprises an accelerometer and the second motion sensor comprises either an optomechanical sensor or a pressure sensor, and wherein the physiological sensor and the at least one motion sensor are in communication with at least one processor (40), the operations comprising:
processing a motion sensor signal received from at least one of the first and second motion sensors (50) to identify an activity characteristic of the subject;
selecting the first motion sensor or the second motion sensor as a noise reference in response to identification of the activity characteristic; and
processing a physiological sensor signal received from the physiological sensor (24, 34) using the noise reference to generate an output signal having reduced noise relative to the physiological sensor signal.

## Patentansprüche

1. Überwachungsvorrichtung (20, 30), die so konfiguriert ist, dass sie an einer Körperstelle eines Probanden angebracht werden kann, wobei die Überwachungsvorrichtung umfasst:
ein Sensormodul (24, 34) mit:
einen oder mehrere physiologische Sensoren, die so konfiguriert sind, dass sie physiologische Daten der Person erfassen und/oder messen und ein dafür repräsentatives physiologisches Sensorsignal erzeugen;
einen ersten und einen zweiten Bewegungssensor (50), die so konfiguriert sind, dass sie Bewegungsdaten der Person erfassen und/oder messen und ein dafür repräsentatives Bewegungssensorsignal erzeugen, wobei der erste Bewegungssensor einen Beschleunigungsmesser und der zweite Bewegungssensor entweder einen optomechanischen Sensor oder einen Drucksensor umfasst; und
mindestens einen Prozessor (40), der mit dem einen oder den mehreren physiologischen Sensoren (24, 34) und dem ersten und zweiten Bewegungssensor (50) gekoppelt ist,
wobei der mindestens eine Prozessor (40) so konfiguriert ist, dass er Operationen ausführt, die Folgendes umfassen:
Verarbeiten des von dem ersten und dem zweiten Bewegungssensor (50) empfangenen Bewegungssensorsignals, um eine Aktivitätscharakteristik des Subjekts zu identifizieren;
Auswählen einer Geräuschreferenz als Reaktion auf die Identifizierung der Aktivitätscharakteristik; und
Verarbeiten des von dem einen oder den mehreren physiologischen Sensoren (24, 34) empfangenen physiologischen Sensorsignals unter Verwendung der Geräuschreferenz, um ein Ausgangssignal zu erzeugen, das im Vergleich zu dem physiologischen Sensorsignal ein reduziertes Rauschen aufweist.

2. Überwachungsvorrichtung nach Anspruch 1, wobei das Auswählen des ersten Bewegungssensors oder des zweiten Bewegungssensors als Geräuschreferenz umfasst:
Auswählen des ersten Bewegungssensors als Reaktion auf die Identifizierung der Aktivitätscharakteristik als periodische Bewegung; oder
Auswählen des zweiten Bewegungssensors als Reaktion auf die Identifizierung der Aktivitätscharakteristik als nicht-periodische Bewegung.

3. Überwachungsvorrichtung nach Anspruch 2, wobei die physiologischen Sensoren (24, 34) einen optischen Sensor umfassen und wobei eine Signalantwort des zweiten Bewegungssensors, enger mit bewegungsbezogenem optischen Rauschen korrespondiert, das in dem physiologischen Sensorsignal vorhanden ist, als eine Signalantwort des ersten Bewegungssensors (50).

4. Überwachungsvorrichtung nach Anspruch 2 oder 3, wobei der eine oder die mehreren physiologischen Sensoren (24, 34) einen Photoplethysmographie-Sensor (PPG-Sensor) umfassen.

5. Überwachungsvorrichtung nach Anspruch 4, wobei der optomechanische Sensor (50) so konfiguriert ist, dass er das von einem optischen Emitter erzeugte Licht in Reaktion auf Körperbewegungen moduliert und das Licht auf einen Weg lenkt, der im Wesentlichen unbeeinflusst von dem Blutfluss ist.

6. Überwachungsvorrichtung nach einem der vorstehenden Ansprüche, wobei die Verarbeitung des physiologischen Sensorsignals unter Verwendung der Rauschreferenz umfasst:
Verarbeiten des physiologischen Sensorsignals unter Verwendung eines frequenzbereichsbasierten Algorithmus oder einer Schaltung, die auf die Identifizierung der Aktivitätscharakteristik als periodische Bewegung reagiert; oder
Verarbeiten des physiologischen Sensorsignals unter Verwendung eines zeitbereichsbasierten Algorithmus oder einer zeitbereichsbasierten Schaltung, die auf die Identifizierung der Aktivitätscharakteristik als nichtperiodische Bewegung reagiert.

7. Überwachungsvorrichtung nach Anspruch 1, wobei die Verarbeitung des
physiologischen Sensorsignals unter Verwendung der Rauschreferenz ferner umfasst:
als Reaktion auf die Identifizierung der Aktivitätscharakteristik als periodische Bewegung, die Verarbeitung des physiologischen Sensorsignals unter Verwendung eines ersten Algorithmus oder einer ersten Schaltung; oder
als Reaktion auf die Identifizierung der Aktivitätscharakteristik als nichtperiodische Bewegung, die Verarbeitung des physiologischen Sensorsignals unter Verwendung eines zweiten Algorithmus oder einer zweiten Schaltung, die sich von dem ersten Algorithmus oder der ersten Schaltung unterscheiden.

8. Überwachungsvorrichtung nach Anspruch 7, wobei der erste und der zweite Algorithmus oder die erste und die zweite Schaltung denselben Sensor oder dieselben Sensoren des mindestens einen Bewegungssensors verwenden.

9. Überwachungsvorrichtung nach Anspruch 7 oder 8, wobei der erste Algorithmus oder die erste Schaltung so konfiguriert ist, dass sie eine Spektralsubtraktion durchführt, und wobei der zweite Algorithmus oder die zweite Schaltung so konfiguriert ist, dass sie eine adaptive Filterung durchführt.

10. Überwachungsvorrichtung nach Anspruch 1, wobei die in der Geräuschreferenz enthaltenen Körperbewegungsinformationen größer sind als die darin enthaltenen Blutflussinformationen.

11. Überwachungsvorrichtung nach Anspruch 1, wobei die Geräuschreferenz Informationen umfasst, die mit der Bewegung des Probanden in Verbindung stehen, und im Wesentlichen frei von Informationen ist, die mit dem Blutfluss des Probanden in Verbindung stehen.

12. Überwachungsvorrichtung nach Anspruch 1, wobei die physiologischen Informationen eine oder mehrere der folgenden Informationen umfassen:
Herzfrequenz des Probanden, Atemfrequenz des Probanden, Atemfrequenzintervall des Probanden, RRi, Herzfrequenzvariabilität des Probanden, HRV, Blutdruck des Probanden, Blutwerte des Probanden, kardiovaskuläre Eigenschaften des Probanden.

13. Überwachungsvorrichtung nach Anspruch 1, wobei die Aktivitätscharakteristik eine erste Aktivitätscharakteristik oder eine zweite Aktivitätscharakteristik umfasst, die sich von der ersten Aktivitätscharakteristik unterscheidet, wobei das Auswählen der Rauschreferenz umfasst:
Auswählen des ersten Bewegungssensors (50) als Reaktion auf die Identifizierung der ersten Aktivitätscharakteristik; oder
Auswählen des zweiten Bewegungssensors (50) als Reaktion auf die Identifizierung der zweiten Aktivitätscharakteristik.

14. Verfahren zum Überwachen einer Person über eine Überwachungsvorrichtung (20, 30), wobei die Überwachungsvorrichtung ein Sensormodul (24, 34) mit einem oder mehreren physiologischen Sensoren, die so konfiguriert sind, dass sie physiologische Daten von der Person erfassen und/oder messen und ein dafür repräsentatives physiologisches Sensorsignal erzeugen, und einen ersten und einen zweiten Bewegungssensor (50), die jeweils so konfiguriert sind, dass sie Bewegungsdaten der Person erfassen und/oder messen und ein dafür repräsentatives Bewegungssensorsignal erzeugen, wobei der erste Bewegungssensor einen Beschleunigungsmesser umfasst und der zweite Bewegungssensor entweder einen optomechanischen Sensor oder einen Drucksensor umfasst, und wobei der physiologische Sensor und der mindestens eine Bewegungssensor mit mindestens einem Prozessor (40) in Verbindung stehen, wobei das Verfahren umfasst:
Verarbeiten eines Bewegungssensorsignals, das von mindestens einem der ersten und zweiten Bewegungssensoren (50) empfangen wird, durch den mindestens einen Prozessor (40), um eine Aktivitätscharakteristik der Person zu identifizieren;
Auswählen des ersten Bewegungssensors oder des zweiten Bewegungssensors als Geräuschreferenz durch den mindestens einen Prozessor (40) als Reaktion auf die Identifizierung der Aktivitätscharakteristik; und
Verarbeiten eines von dem physiologischen Sensor (24, 34) empfangenen physiologischen Sensorsignals durch den mindestens einen Prozessor (40) unter Verwendung der Geräuschreferenz, um ein Ausgangssignal zu erzeugen, das im Vergleich zum physiologischen Sensorsignal ein reduziertes Rauschen aufweist.

15. Computerprogrammprodukt, das ein nicht-transitorisches, computerlesbares Speichermedium (60) umfasst, in dem ein computerlesbarer Programmcode enthalten ist, der, wenn er von mindestens einem Prozessor (40) ausgeführt wird, den mindestens einen Prozessor dazu veranlasst, Operationen für ein Verfahren zur Überwachung eines Subjekts über eine Überwachungsvorrichtung (20, 30) durchzuführen, wobei die Überwachungsvorrichtung ein Sensormodul (24, 34) mit einem oder mehreren physiologischen Sensoren, die so konfiguriert sind, dass sie physiologische Daten von der Person erfassen und/oder messen und ein dafür repräsentatives physiologisches Sensorsignal erzeugen, und einen ersten und einen zweiten Bewegungssensor (50), die jeweils so konfiguriert sind, dass sie Bewegungsdaten der Person erfassen und/oder messen und ein dafür repräsentatives Bewegungssensorsignal erzeugen, wobei der erste Bewegungssensor einen Beschleunigungsmesser umfasst und der zweite Bewegungssensor entweder einen optomechanischen Sensor oder einen Drucksensor umfasst, und wobei der physiologische Sensor und der mindestens eine Bewegungssensor mit mindestens einem Prozessor (40) in Verbindung stehen, wobei die Vorgänge Folgendes umfassen:
Verarbeiten eines Bewegungssensorsignals, das von mindestens einem der ersten und zweiten Bewegungssensoren (50) empfangen wird, um eine Aktivitätscharakteristik der Person zu identifizieren;
Auswählen des ersten Bewegungssensors oder des zweiten Bewegungssensors als Geräuschreferenz als Reaktion auf die Identifizierung der Aktivitätscharakteristik; und
Verarbeiten eines von dem physiologischen Sensor (24, 34) empfangenen physiologischen Sensorsignals unter Verwendung der Geräuschreferenz, um ein Ausgangssignal zu erzeugen, das im Vergleich zum physiologischen Sensorsignal ein reduziertes Rauschen aufweist.

## Revendications

1. Un dispositif de surveillance (20, 30) configuré pour être fixé à un emplacement corporel d'un sujet, le dispositif de surveillance comprenant :
un module capteur (24, 34) avec :
un ou plusieurs capteurs physiologiques configurés pour détecter et/ou mesurer des données physiologiques en provenance du sujet et pour générer un signal de capteur physiologique représentatif de celles-ci ;
un premier et un second capteur de mouvement (50) configurés pour détecter et/ou mesurer des données de mouvement du sujet et générer un signal de capteur de mouvement représentatif de celles-ci, le premier capteur de mouvement comprenant un accéléromètre et le second capteur de mouvement comprenant soit un capteur optomécanique soit un capteur de pression ; et
au moins un processeur (40) couplé aux un ou plusieurs capteurs physiologiques (24, 34) et au premier et au second capteur de mouvement (50), dans lequel
l'au moins un processeur (40) est configuré pour effectuer des opérations comprenant :
le traitement du signal de capteur de mouvement reçu en provenance du premier et du second capteur de mouvement (50) pour identifier une caractéristique d'activité du sujet ;
la sélection d'une référence de bruit en réponse à l'identification de la caractéristique d'activité ; et
le traitement du signal de capteur physiologique reçu en provenance des un ou plusieurs capteurs physiologiques (24, 34) en utilisant la référence de bruit pour générer un signal de sortie avec un bruit réduit par rapport au signal de capteur physiologique.

2. Le dispositif de surveillance de la revendication 1, dans lequel la sélection du premier capteur de mouvement ou du second capteur de mouvement en tant que référence de bruit comprend :
la sélection du premier capteur de mouvement en réponse à l'identification de la caractéristique d'activité comme étant un mouvement périodique ; ou
la sélection du second capteur de mouvement en réponse à l'identification de la caractéristique d'activité comme étant un mouvement non périodique.

3. Le dispositif de surveillance de la revendication 2, dans lequel les capteurs physiologiques (24, 34) comprennent un capteur optique, et dans lequel une réponse de signal du second capteur de mouvement correspond d'une façon plus proche à un bruit optique en relation avec le mouvement, présent dans le signal de capteur physiologique, qu'une réponse de signal du premier capteur de mouvement (50).

4. Le dispositif de surveillance des revendications 2 ou 3, dans lequel les un ou plusieurs capteurs physiologiques (24, 34) comprennent un capteur photopléthysmographique, PPG.

5. Le dispositif de surveillance de la revendication 4, dans lequel le capteur optomécanique (50) est configuré pour moduler la lumière générée par un émetteur optique en réponse à un mouvement du corps, et pour diriger la lumière suivant un trajet qui est substantiellement non affecté par le flux sanguin.

6. Le dispositif de surveillance de l'une des revendications précédentes, dans lequel le traitement du signal de capteur physiologique en utilisant la référence de bruit comprend :
le traitement du signal de capteur physiologique en utilisant un algorithme ou un circuit basés dans le domaine fréquentiel, qui répondent à une identification de la caractéristique d'activité comme étant un mouvement périodique ; ou
le traitement du signal de capteur physiologique en utilisant un algorithme ou un circuit basés dans le domaine temporel, qui répondent à une identification de la caractéristique d'activité comme étant un mouvement non périodique.

7. Le dispositif de surveillance de la revendication 1, dans lequel le traitement du signal de capteur physiologique en utilisant la référence de bruit comprend en outre :
en réponse à l'identification de la caractéristique d'activité comme étant un mouvement périodique, le traitement du signal de capteur physiologique en utilisant un premier algorithme ou circuit ; ou
en réponse à l'identification de la caractéristique d'activité comme étant un mouvement non périodique, le traitement du signal de capteur physiologique en utilisant un second algorithme ou circuit qui sont différents du premier algorithme ou circuit.

8. Le dispositif de surveillance de la revendication 7, dans lequel le premier et le second algorithme ou circuit utilisent un même capteur ou capteurs de l'au moins un capteur de mouvement.

9. Le dispositif de surveillance de la revendication 7 ou 8, dans lequel le premier algorithme ou circuit est configuré pour effectuer une soustraction spectrale, et dans lequel le second algorithme ou circuit est configuré pour effectuer un filtrage adaptatif.

10. Le dispositif de surveillance de la revendication 1, dans lequel les informations de mouvement du corps contenues dans la référence de bruit sont plus importantes que les informations de flux sanguin contenues dans celles-ci.

11. Le dispositif de surveillance de la revendication 1, dans lequel la référence de bruit comprend des informations associées au mouvement du sujet et est substantiellement dépourvue d'informations associées au flux sanguin du sujet.

12. Le dispositif de surveillance de la revendication 1, dans lequel les informations physiologiques comprennent une ou plusieurs des informations suivantes : rythme cardiaque du sujet, rythme respiratoire du sujet, intervalle de rythme respiratoire, RRI, du sujet, variabilité du rythme cardiaque, HRV, du sujet, pression sanguine du sujet, niveau d'analyte sanguin du sujet, propriétés cardio-vasculaires du sujet.

13. Le dispositif de surveillance de la revendication 1, dans lequel la caractéristique d'activité comprend une première caractéristique d'activité ou une seconde caractéristique d'activité qui est différente de la première caractéristique d'activité, dans lequel la sélection de la référence de bruit comprend :
la sélection du premier capteur de mouvement (50) en réponse à l'identification de la première caractéristique d'activité ; ou
la sélection du second capteur de mouvement (50) en réponse à l'identification de la seconde caractéristique d'activité.

14. Un procédé de surveillance d'un sujet via un dispositif de surveillance (20, 30), dans lequel le dispositif de surveillance comprend un module capteur (24, 34) avec un ou plusieurs capteurs physiologiques configurés pour détecter et/ou mesurer des données physiologiques en provenance du sujet et pour générer un signal de capteur physiologique représentatif de celles-ci, et un premier et un second capteur de mouvement (50) configurés chacun pour détecter et/ou mesurer des données de mouvement du sujet et pour générer un signal de capteur de mouvement représentatif de celles-ci, le premier capteur de mouvement comprenant un accéléromètre et le second capteur de mouvement comprenant soit un capteur optomécanique soit un capteur de pression, et le capteur physiologique et l'au moins un capteur de mouvement étant en communication avec au moins un processeur (40), le procédé comprenant :
le traitement d'un signal de capteur de mouvement reçu en provenance d'au moins l'un du premier et du second capteur de mouvement (50) par l'au moins un processeur (40) pour identifier une caractéristique d'activité du sujet ;
la sélection du premier capteur de mouvement ou du second capteur de mouvement en tant que référence de bruit par l'au moins un processeur (40) en réponse à l'identification de la caractéristique d'activité ; et
le traitement d'un signal de capteur physiologique reçu en provenance du capteur physiologique (24, 34) par l'au moins un processeur (40) en utilisant la référence de bruit pour générer un signal de sortie présentant un bruit réduit par rapport au signal de capteur physiologique.

15. Un produit de programme de calculateur comprenant un support de stockage non transitoire lisible par calculateur (60) avec incorporé dedans du code de programme lisible par calculateur qui, lorsqu'il est exécuté par au moins un processeur (40), fait en sorte que l'au moins un processeur effectue des opérations pour un procédé de surveillance d'un sujet via un dispositif de surveillance (20, 30), le dispositif de surveillance comprenant un module capteur (24, 34) avec un ou plusieurs capteurs physiologiques configurés pour détecter et/ou mesurer des données physiologiques en provenance du sujet et pour générer un signal de capteur physiologique représentatif de celles-ci, et un premier et un second capteur de mouvement (50) configurés chacun pour détecter et/ou mesurer des données de mouvement du sujet et pour générer un signal de capteur de mouvement représentatif de celles-ci, le premier capteur de mouvement comprenant un accéléromètre et le second capteur de mouvement comprenant soit un capteur optomécanique soit un capteur de pression, et le capteur physiologique et l'au moins un capteur de mouvement étant en communication avec au moins un processeur (40), les opérations comprenant :
le traitement d'un signal de capteur de mouvement reçu en provenance d'au moins l'un du premier et du second capteur de mouvement (50) pour identifier une caractéristique d'activité du sujet ;
la sélection du premier capteur de mouvement ou du second capteur de mouvement en tant que référence de bruit par l'au moins un processeur (40) en réponse à l'identification de la caractéristique d'activité ; et
le traitement d'un signal de capteur physiologique reçu en provenance du capteur physiologique (24, 34) en utilisant la référence de bruit pour générer un signal de sortie présentant un bruit réduit par rapport au signal de capteur physiologique.
